# EUROPÄISCHE PATENTSCHRIFT

(11) **EP 1 909 905 B1**
(45) Veröffentlichungstag und Bekanntmachung des Hinweises auf die Patenterteilung: **23.03.2011**
(21) Anmeldenummer: 06762723.2
(22) Anmeldetag: 20.07.2006
(51) Int. Cl.: A61N 5/10

(54) **BESTRAHLUNGSEINRICHTUNG**
IRRADIATION DEVICE
DISPOSITIF DE RAYONNEMENT

(30) Priorität: 22.07.2005 DE 102005035141
(43) Veröffentlichungstag der Anmeldung: 16.04.2008
(73) Patentinhaber: GSI Helmholtzzentrum für Schwerionenforschung GmbH, 64291 Darmstadt (DE)
(72) Erfinder: FEHRENBACHER, Georg, 64367 Mühltal (DE); RADON, Torsten, 61239 Ober-Mörlen (DE)
(74) Vertreter: Mergel, Volker
(86) Internationale Anmeldenummer: PCT/EP2006/007131
(87) Internationale Veröffentlichungsnummer: WO 2007/009786

(56) Entgegenhaltungen:
- EP-A- 1 430 932
- DE-A1- 10 010 523
- DE-A1- 10 235 116
- DE-A1-102004 029 026
- NL-C2- 1 009 865
- US-A- 5 349 198
- KRAFT G ET AL: "THE DARMSTADT PROGRAM HITAG: HEAVY ION THERAPY AT GSI" HADRONTHERAPY IN ONCOLOGY: PROCEEDINGS OF THE INTERNATIONAL SYMPOSIUM ON HADRONTHERAPY, 1994, Seiten 217-228, XP000949119
- AGOSTEO S ET AL: "Shielding calculations for a 250 MeV hospital-based proton accelerator" NUCLEAR INSTRUMENTS & METHODS IN PHYSICS RESEARCH, SECTION - A: ACCELERATORS, SPECTROMETERS, DETECTORS AND ASSOCIATED EQUIPMENT, ELSEVIER, AMSTERDAM, NL, Bd. A374, Nr. 2, 21. Mai 1996 (1996-05-21), Seiten 254-268, XP004007541 ISSN: 0168-9002

## Beschreibung

Die Erfindung betrifft eine Bestrahlungseinrichtung für die Protonen- und/oder Ionenstrahltherapie gemäß Anspruch 1. Derartige auch als Therapieanlagen bezeichnete Einrichtungen sind bekannt. Sie weisen eine in der Regel auf der Basis eines Zyklotrons oder Synchrotons realisierte Strahlungsquelle mit einer Strahlführungseinrichtung sowie mindestens einen Therapieraum auf, in den ein Therapiestrahl eingeleitet wird. Dieser wird auf einen Behandlungsort gerichtet an, dem ein zu behandelnder Patient platziert werden kann.

Bei der Beschleunigung von Strahlen und bei deren Nutzung während der Patientenbestrahlung wird Sekundärstrahlung erzeugt. Beim Abbremsen von Ionen werden in dem hier zu betrachtenden Energiebereich bis zu einigen 100 MeV pro Nukleon in Kernreaktionen (Spallationsreaktionen oder Fragmentierungen der Projektil- oder Targetkerne) Neutronen, Protonen, leichte Ionen und Gammastrahlung erzeugt. Die Abschirmung der Sekundärstrahlung wird im Wesentlichen durch die produzierte Neutronenstrahlung dominiert.

Es ist bekannt, dass der größte Teil eines beschleunigten Therapiestrahls im Gewebe deponiert wird und dort einen - in Richtung des Therapiestrahls gesehen - stark nach vorne gerichteten Kegel von Neutronenstrahlung erzeugt. Die meisten der erzeugten Neutronen verlassen den Patienten ohne Wechselwirkung. Da die Neutronenstrahlung sehr hohe Energien haben kann und die Zehntelwertschichtdicke für hochenergetische Neutronenstrahlung z.B. in Normalbeton einen Meter beträgt, ist gerade in Vorwärtsrichtung des einfallenden Therapiestrahls ein erhebliche Abschirmaufwand nötig. Die bauliche Strahlenschutzauslegung muss diesen strahlenphysikalischen und geometrischen Randbedingungen Rechnung tragen.

Bei der Planung von Behandlungszentren, insbesondere Klinikanlagen für die Teilchentherapie, in denen Bestrahlungseinrichtungen der hier angesprochenen Art verwendet werden, tritt häufig das Problem auf, dass diese Anlage in einer vorhandenen Infrastruktur in der Nähe oder innerhalb eines größeren Behandlungszentrums gebaut werden muss. Teilchentherapieanlagen mit Zyklotrons und Synchrotrons haben aufgrund der aufwändigen Beschleunigertechnik und den zugehörigen Strahlführungseinrichtungen einen großen Flächenbedarf. Zur Bereitstellung der Therapiestrahlen bedarf es oft eines verzweigten Systems von Strahlführungen, um die Strahlen der Strahlungsquelle Behandlungsräumen zuzuführen. Dadurch ist der Zugang zu diesen Räumen häufig eingeschränkt. Oft lässt es sich nicht vermeiden, dass der Zugang zu einem Therapieraum im Bereich der Neutronenstrahlung der Therapieeinheit angeordnet ist, sodass häufig schwere Abschirmtüren installiert werden müssen, die einerseits den Raumbedarf erhöhen, anderseits den Zugang zum Patienten verzögern.

Aufgabe der Erfindung ist es daher, eine Bestrahlungseinrichtung zu schaffen, welche die hier genannten Nachteile vermeidet.

Zur Lösung dieser Aufgabe wird eine Bestrahlungseinrichtung für die Protonen- und/oder Ionenstrahltherapie mit einer Strahlungsquelle vorgeschlagen, die die in Anspruch 1 genannten Merkmale aufweist. Sie umfasst eine Strahlführungseinrichtung und mindestens einen Behandlungsort und einen Zugang aufweisenden Therapieraum, in den ein Therapiestrahl zur Behandlung eines Patienten eingeleitet wird. Die Bestrahlungseinrichtung zeichnet sich dadurch aus, dass verschiedene Ebenen vorgesehen sind. Dabei ist der Therapieraum in einer ersten Ebene angeordnet. Der Therapiestrahl wird aus einer ober- oder unterhalb dieser Ebene liegenden zweiten Ebene in den Therapieraum eingeleitet und auf den Behandlungsort so gerichtet, dass der Therapiestrahl vom Zugang weggerichtet ist. Dadurch, dass Teile der Bestrahlungseinrichtung auf unterschiedlichen Ebenen angeordnet werden, kann der Raumbedarf deutlich reduziert werden. Außerdem kann die Strahlführungseinrichtung aus der Ebene herausverlagert werden, in der der Therapieraum angeordnet ist. Damit kann der Zugang zum Therapieraum unabhängig von der Strahlführung gewählt werden. Bei der erfindungsgemäßen Bestrahlungseinrichtung ist vorgesehen, dass im Therapieraum eine zum Behandlungsort offene Abschirmung vorgesehen ist. Diese schützt die weiteren Bereiche des Therapieraums vor der Strahlung. Der Zugang zum Therapieraum wird auf der dem Behandlungsort abgewandten Seite der Abschirmung angeordnet, sodass seitlich versetzt zu dem im Therapieraum verlaufenden Therapiestrahl und zur Abschirmung mindestens ein vom Zugang zum Behandlungsort führendes Labyrinth vorgesehen werden kann. Dieses Labyrinth wird von dem Therapiestrahl nicht getroffen. Auch die Neutronenstrahlung, die strahlabwärts vom Behandlungsort entstehen kann, trifft das Labyrinth nicht. Der Zugang zu dem Therapieraum kann also allein durch das Labyrinth vor reflektierten Strahlen geschützt werden, insbesondere können schwere Abschirmtüren entfallen. Damit ergibt sich ein einfacher und rascher Zugang zum Behandlungsort.

Dadurch, dass die im Therapieraum im Bereich des Behandlungsorts entstehende Neutronenstrahlung gerade nicht den Zugang und das Labyrinth zwischen Zugang und Behandlungsort trifft, wie dies bei dem Therapieraum gemäß der DE 102 35 116 A1 der Fall ist, kann der Abschirmungsaufwand reduziert werden. Dies verringert den Raumbedarf der Bestrahlungseinrichtung.

Das Dokument von Kraft G et al: "THE DARMSTADT PROGRAM HITAG: HEAVY ION THERAPY AT GSI", HADRONTHERAPY IN ONCOLOGY, PROCEEDINGS OF THE INTERNATIONAL SYMPOSIUM ON HADRONTHERAPY, 1994, Seiten 217-228, XP000949119, offenbart eine Bestrahlungseinrichtung für die Ionenstrahltherapie mit einer Strahlungsquelle, einer Strahlführungseinrichtung und mit einem, wenigstens einen Behandlungsort und einen Zugang umfassenden Therapieraum, in den ein Therapiestrahl eingeleitet wird, wobei der Therapieraum in einer ersten Ebene angeordnet ist, der Therapiestrahl in den Therapieraum eingeleitet, dort umgelenkt und auf den Behandlungsort so gerichtet wird, dass der auf den Behandlungsort gerichtete Therapiestrahl vom Zugang weggerichtet ist, im Therapieraum eine in Richtung auf den Behandlungsort offene Abschirmung vorgesehen ist, der Zugang auf der dem Behandlungsort abgewandten Seite der Abschirmung angeordnet ist, und seitlich versetzt zu dem im Therapieraum verlaufenden Therapiestrahl und zur Abschirmung mindestens ein vom Zugang zum Behandlungsort führendes Labyrinth vorgesehen ist.

Bei einem bevorzugten Ausführungsbeispiel der Bestrahlungseinrichtung ist vorgesehen, dass zwischen dem Zugang und dem Behandlungsort auf beiden Seiten der Abschirmung ein Labyrinth vorgesehen ist. Eines der beiden kann als Zugang und das andere als Ausgang verwendet werden, sodass die Nutzung des Therapieraums optimiert werden kann: Der Abtransport von Patienten aus dem Therapieraum behindert den Zugang nicht, sodass ein rascher Patientenwechsel möglich ist.

Ein weiteres bevorzugtes Ausführungsbeispiel der Bestrahlungseinrichtung zeichnet sich dadurch aus, dass Räume, die der Vor- und Nachbehandlung von Patienten beziehungsweise für den Aufenthalt von sonstigen Personen ebenfalls auf der ersten Ebene angeordnet sein können, in der der Therapieraum liegt. Dies ermöglicht eine Optimierung des Ablaufs zur Therapievorbereitung (Computertomographie, Röntgen zur Lageverifizierung usw.) und zur Durchführung der Therapie.

Ein weiteres Ausführungsbeispiel der Bestrahlungseinrichtung zeichnet sich dadurch aus, dass der in den Therapieraum eintretende Therapiestrahl zwar auf den Behandlungsort, nicht aber auf die genannten Räume gerichtet ist. Diese sind also keinesfalls durch den Therapiestrahl und die bei der Behandlung produzierte Neutronenstrahlung belastet. Dabei bedarf es nicht einmal einer besonderen Abschirmung zwischen dem Eintrittsbereich und den genannten Räumen, weil der Therapieraum so angeordnet werden kann, dass die Neutronenstrahlung beispielsweise ins Erdreich geleitet wird, wo eine Belastung von Patienten, Behandlungspersonal und von Leuten vermieden wird, die die Bestrahlungseinrichtung bedienen und warten.

Bei einem weiteren bevorzugten Ausführungsbeispiel der Bestrahlungseinrichtung ist vorgesehen, dass die Strahlungsquelle in einer dritten Ebene angeordnet ist. Dadurch, dass die Bestrahlungseinrichtung auf mehrere Ebenen verteilt ist, und dass hier eine weitere Ebene eröffnet wird, können die einzelnen Elemente wie Strahlungsquelle, Strahlführungseinrichtung und Therapieräume quasi übereinander verschachtelt werden, um den Raumbedarf zu minimieren. Dabei bleibt vorgesehen, dass der auf den Behandlungsort gerichtete Strahl von den Räumen weggerichtet ist, in denen Patienten und sonstige Personen sich aufhalten können.

Weitere Ausgestaltungen ergeben sich aus den übrigen Unteransprüchen.

Die Erfindung wird im Folgenden anhand einer Zeichnung näher erläutert. Es zeigen:
- Figur 1: eine Prinzipskizze einer Bestrahlungseinrichtung im Längsschnitt;
- Figur 2: eine Draufsicht auf die in Figur 2 dargestellte Bestrahlungseinrichtung,
- Figur 3: ein erstes Ausführungsbeispiel eines Therapieraums in Draufsicht;
- Figur 4: ein abgewandeltes Ausführungsbeispiel eines in Figur 3 dargestellten Therapieraums in Draufsicht;
- Figur 5: ein weiteres Ausführungsbeispiel eines Therapieraums in Draufsicht;
- Figur 6: eine Anzahl von Therapieräumen gemäß Figur 3 in bogenförmiger Anordnung;
- Figur 7: eine Anzahl von Therapieräumen gemäß Figur 4 in bogenförmiger Anordnung;
- Figur 8: eine Anzahl von Therapieräumen gemäß Figur 4 in paralleler Anordnung und
- Figur 9: eine Anzahl von Therapieräumen gemäß Figur 4 in spiegelbildlicher Anordnung.

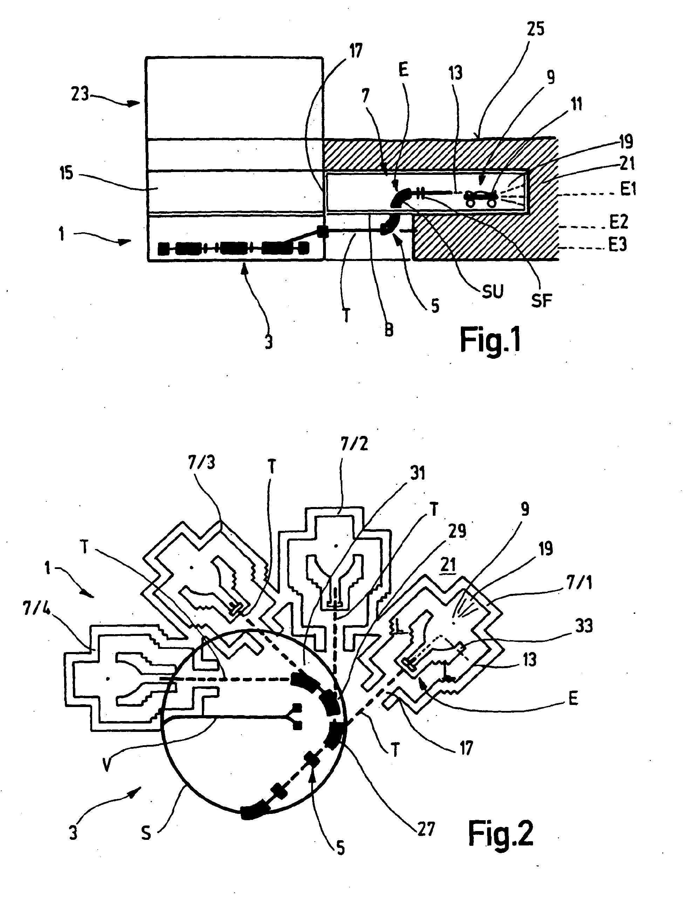

Der Prinzipskizze gemäß Figur 1 ist eine Bestrahlungseinrichtung 1 zu entnehmen, die eine Strahlungsquelle 3, eine Strahlführungseinrichtung 5 und einen Therapieraum 7 mit einem Behandlungsort 9 umfasst, an dem ein Patient 11 einem Therapiestrahl 13 aussetzbar ist. Der Prinzipskizze ist zu entnehmen, dass sich der Therapieraum 7 auf einer ersten Ebene E1 befindet, während die Strahlführungseinrichtung 5 auf einer zweiten Ebene E2 und die Strahlungsquelle 3 auf einer dritten Ebene E3 angeordnet sind, wobei die Ebenen E2 und E3 hier unterhalb der ersten Ebene E1 angeordnet sind. Es ist aber sehr wohl denkbar, dass die Strahlführungseinrichtung 5 und der Therapieraum 7 unterhalb der Strahlungsquelle 3 angeordnet werden können.

Aus der Prinzipskizze gemäß Figur 1 ist ersichtlich, dass der die Strahlführungseinrichtung 5 verlassende Strahl in einem Eintrittsbereich E in den Therapieraum 7 eintritt.

Figur 1 zeigt noch, dass oberhalb der Strahlführungseinrichtung 5 im Therapieraum 7 eine Strahlumlenkungseinrichtung SU sowie eine Strahlformuhgseinrichtung SF vorgesehen sind.

Neben dem Therapieraum 7 ist ein Raum 15 angedeutet, der der Patientenvorbereitung dienen kann, und in dem sich Bedienungspersonal für die Bestrahlungseinrichtung 1 aufhalten kann.

Der Darstellung ist zu entnehmen, dass die Strahlungsquelle 3, die Strahlführungseinrichtung 5 und der Therapieraum 7, sowie weitere der Bestrahlungseinrichtung 1 zugeordnete Räume, hier der Raum 15, übereinander angeordnet sein können, was zu einer deutlichen Raumeinsparung führt. Überdies zeigt sich, dass die Strahlführungseinrichtung 5 hier unterhalb des Therapieraums 7 angeordnet ist und damit den Zugang zum Therapieraum 7 in keiner Weise stört. Bei der Bestrahlungseinrichtung 1 gemäß Figur 1 ist vorgesehen, dass der Zugang 17 sich im Übergangsbereich zwischen dem Raum 15 und dem Therapieraum 7 befindet. Bei der Darstellung gemäß Figur 1 betritt man also den Therapieraum 7 aus dem Raum 15, indem man von links nach rechts durch den Zugang 17 geht. Entsprechend gelangt man vom Therapieraum 7 bei der Darstellung gemäß Figur 1 von rechts nach links in den Raum 15. Der Eintrittsbereich E liegt innerhalb des Therapieraums 7 in einem Abstand rechts vom Zugang 17 und in einem Abstand links vom Behandlungsort 9.

Dadurch, dass der Therapiestrahl 13 den Therapieraum 7 ausgehend vom Eintrittsbereich E von links nach rechts durchläuft, wird der Zugang 17 in keinem Fall von dem Therapiestrahl 13 betroffen. Auch jenseits des Behandlungsorts 9 gegebene Neutronenstrahlung 19 trifft keinesfalls auf den Zugang 17. Sie verläuft vom Eintrittsbereich E gesehen rechts und wird vielmehr in das sich an den Therapieraum 7 anschließende Erdreich 21 geleitet, das rauch oberhalb des Therapieraums 7 vorgesehen ist. Es wird also deutlich, dass - in Richtung des Therapiestrahls 13 gesehen - jenseits des Behandlungsorts 9 keine besondere Abschirmung mehr vorzusehen ist, weil, wie gesagt, die Neutronenstrahlung 19 in das Erdreich 21 abgeführt wird und damit keinesfalls zu dem Raum 15 gelangen kann, der - in Richtung des Therapiestrahls 13 gesehen - an der gegenüberliegenden Seite des Therapieraums 7 zum Behandlungsort 9 angeordnet ist.

Die Figur zeigt auch deutlich, dass der Zugang 17 weder mit dem Therapiestrahl 13 noch mit der Neutronenstrahlung 19 beaufschlagt wird.

Der Seitenansicht ist zu entnehmen, dass die Strahlführungseinrichtung 5 so ausgebildet ist, dass ein Teilstrahl T der Strahlungsquelle 3 von unten durch den Boden B des Therapieraums 7 im Eintrittsbereich E in den Therapieraum eingeleitet und dort so umgelenkt wird, dass er parallel zum Boden B, also horizontal in Richtung auf den Behandlungsort 9 verläuft.

Die Umlenkung des Teilstrahls T kann auch so gewählt werden, dass dieser unter einem Winkel in den Therapieraum 7 eintritt, oder aber so, dass er innerhalb des Therapieraumes 7 so umgelenkt wird, dass er nicht parallel zum Boden B sondern unter einem Winkel zu diesem verläuft. Damit verläuft der Therapiestrahl 13 auch im Behandlungsort 9 unter einem Winkel zum Boden B.

Es zeigt sich also, dass die Bestrahlungseinrichtung 1 auch in diesem Gesichtspunkt variabel ausgestaltet sein kann. Dabei ist es auch möglich, dass der Therapiestrahl 13 unter einem Winkel durch die Decke die. Therapieraums 7 in diesen eintritt oder dort so umgelenkt wird, dass er nicht parallel zum Boden B sondern von schräg oben auf den Behandlungsort 9 trifft. Dieser Strahlungsverlauf hat den Vorteil, dass der Therapiestrahl 13 und die Neutronenstrahlung 19 von oben in das Erdbereich 21 geleitet werden, sodass eine optimale Absorption sichergestellt ist. Bei einer Strahlungsrichtung schräg nach oben müsste sichergestellt werden, dass das Erdreich 21 über dem Therapieraum 7 dick genug ist oder dass dort zusätzlich eine Abschirmung bereitgestellt wird.

Dadurch, dass hier auch die Möglichkeit geschaffen wird, den Teilstrahl T und insbesondere den Therapiestrahl 13 unter einem Winkel auf den Behandlungsort 9 zu richten, kann in einigen Fällen auf eine Gantry verzichtet werden.

Die Darstellung zeigt auch, dass der Eintrittsbereich E des Teilstrahls T in den Therapieraum 7 in einem Abstand zum Zugang 17 in den Therapieraum 7 und in einem Abstand zum Bestrahlungsort 9 liegt. Besonders deutlich wird dabei, dass der Bereich zwischen dem Zugang 17 und dem Eintrittsbereich E frei ist, weil die Strahlführungseinrichtung 5 hier unterhalb des Bodens B angeordnet ist.

In Figur 1 ist angedeutet, dass der Raum 15 Teil eines Behandlungszentrums, eines Forschungszentrums oder einer Klinik 23 sein kann.

Auch oberhalb des Therapieraums 7 befindet sich Erdreich 21. Durch die Anordnung unterhalb des Erdniveaus 25 kann auch die Abschirmung durch Beton und sonstige Materialien oberhalb des Therapieraums 7 auf ein Minimum reduziert werden.

Insgesamt können hohe Abschirmmassen innerhalb der Bestrahlungseinrichtung 1 und der Klinik 23 vermieden werden, weil natürliche Abschirmmaterialien, nämlich Erdreich 21, eingesetzt werden können, was zusätzlich Kosten in der Einrichtung, aber auch im Rückbau reduziert.

Insgesamt ergeben sich also folgende Vorteile:
Durch die Anordnung der Elemente der Bestrahlungseinrichtung 1 auf verschiedenen Ebenen E1, E2 und E3 ist es insbesondere möglich, die Strahlführungseinrichtung 5 in einer anderen Ebene als den Therapieraum 7 vorzusehen, sodass der Zugang 17 zu diesem und Räume 15, die in Zusammenhang mit der Nutzung des Therapieraums 7 nötig sind, optimal angeordnet werden können, und der Ablauf zur Therapievorbereitung und zur Durchführung der Therapie ungestört ist. Außerdem ist der bauliche Strahlenschutz für die Strahlungsquelle 3, die Strahlführungseinrichtung 5 und für den Therapieraum 7 gewährleistet. Abschirmmaterialien werden sehr effizient verwendet. Insbesondere können teure Materialien wie Beton und dergleichen durch Erdreich ersetzt werden. Außerdem können die einzelnen Elemente der Bestrahlungseinrichtung 1 kompakt angeordnet und die Raumnutzung optimiert werden. Im Übrigen ist die Anknüpfung an vorhandene Behandlungs- und Forschungszentren, insbesondere Kliniken möglich, ohne dass es dort zu zusätzlichen Strahlenbelastungen durch Therapiestrahlen oder Neutronenstrahlung käme.

Figur 2 zeigt die in Figur 1 wiedergegebene Bestrahlungseinrichtung 1 in Draufsicht. Deutlich erkennbar ist hier die Strahlungsquelle 3, die einen auch als LINAC bezeichneten Vorbeschleuniger V und ein Synchrotron S umfasst. Die Strahlungsquelle 3 befindet sich in der untersten Ebene E3.

Oberhalb dieser Ebene befindet sich in der Ebene E2 die Strahlführungseinrichtung 5 mit mehreren Strahlverzweigungen 27, 29 und 31. Oberhalb der Ebene E2 befinden sich hier vier angedeutete Therapieräume 7/1, 7/2, 7/3 und 7/4.

Aus der Darstellung gemäß Figur 2 wird deutlich, dass, von der Strahlungsquelle 3 aus gesehen, jenseits der Strahlverzweigungen 27, 29 und 31 Teilstrahlen T gegeben sind, die den einzelnen Therapieräumen 7/1 bis 7/4 zugeleitet werden.

Da die Therapieräume hier identisch ausgebildet sind, wird im Folgenden nur noch ein Therapieraum 7 beschrieben. Die weiteren Erläuterungen werden anhand des in Figur 2 ganz rechts dargestellten Therapieraums 7/1 erfolgen; er wird im Folgenden allgemein als Therapieraum 7 bezeichnet.

Der Zugang 17 zu dem Therapieraum 7 ist so ausgerichtet, dass er in Richtung des Teilstrahls T weist, wobei aus den Erläuterungen zu Figur 1 deutlich ist, dass der Teilstrahl T unterhalb des Bodens B eines Therapieraums 7 verläuft und erst im Eintrittsbereich E - hier von unten - in den Therapieraum 7 eintritt. Es wird ohne Weiteres deutlich, dass eine derartige Strahleinleitung in einen Therapieraum auch von oben erfolgen kann.

In der Darstellung gemäß Figur 2 ist das Isozentrum am Behandlungsort 9 durch einen Punkt angedeutet. Auf dieses ist der im Eintrittsbereich E in den Therapieraum 7 eintretende Therapiestrahl 13 gerichtet. Jenseits des Behandlungsorts 9 gegebene Neutronenstrahlung 19 ist vom Zugang 17 weggerichtet und tritt in das den Therapieraum 7 umgebende Erdreich 21 ein. Deutlich wird auch hier, dass der Zugang 17 weder durch den Therapiestrahl 13 noch durch die Neutronenstrahlung 19 beaufschlagt wird.

Der Eintrittsbereich E ist von einer in Richtung auf den Behandlungsort 9 offenen, quasi U-förmigen Abschirmung 33 umgeben. Vom Zugang 17 in Richtung zum Behandlungsort 9 gesehen, befindet sich neben der Abschirmung 33 mindestens ein Labyrinth L. Bei dem in Figur 2 dargestellten Ausführungsbeispiel sind rechts und links von der Abschirmung 33 je ein Labyrinth L vorgesehen, von denen eines als Zugang und das andere als Ausgang verwendbar ist, um den Behandlungsablauf zu optimieren.

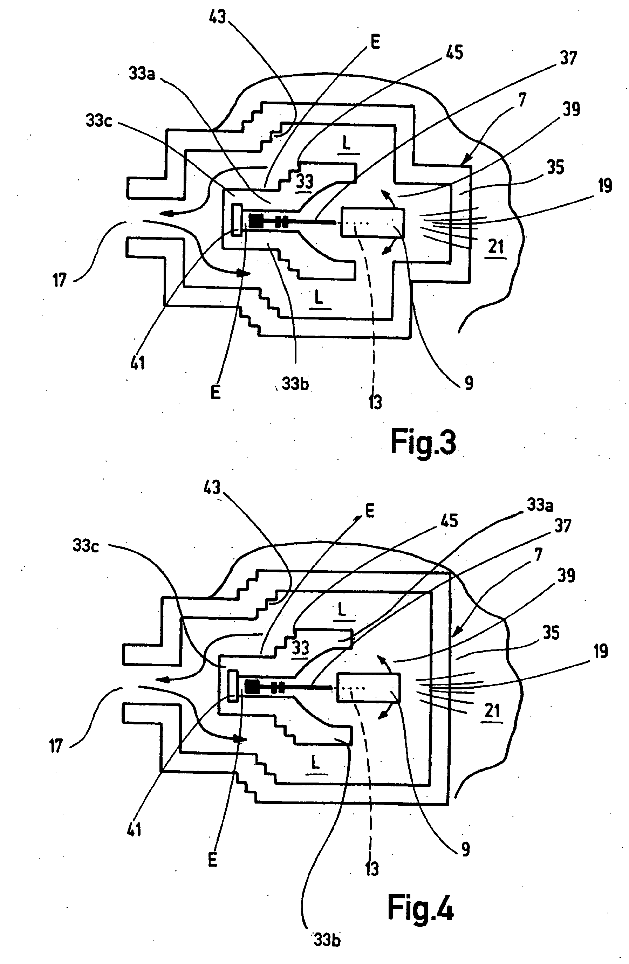

Figur 3 zeigt einen aus Figur 2 ersichtlichen Therapieraum 7 in vergrößerter Darstellung. Gleiche Teile sind mit gleichen Bezugsziffern versehen, sodass insofern auf die vorangegangene Beschreibung verwiesen wird.

Der Therapieraum 7 wird von einer herkömmlichen Abschirmwand 35 umgeben, beispielsweise von einer einen Meter dicken Betonwand. Die Dicke der Wand 35 kann der verschiedenen Anwendungsfälle angepasst werden; ebenso das Abschirmmaterial. So ist es beispielsweise aus der DE 103 12 271 A1 bekannt, Gips zur Abschirmung einzusetzen. Außerhalb des Therapieraums 7 findet sich Erdreich 21.

Bei der Darstellung gemäß Figur 3 ist links der Zugang 17 des Therapieraums 7 gegeben. Im Inneren desselben ist in einem Abstand zum Zugang 17 der Eintrittsbereich E des in den Figuren 1 und 2 dargestellten Teilstrahls T zu sehen. Dieser ist von einer praktisch mitten im Therapieraum 7 gelegenen Abschirmung 33 umgeben.

Aus der Prinzipskizze gemäß Figur 3 ist, wie bereits aus Figur 1, ersichtlich, dass man den Therapieraum 7 durch den Zugang 17 betreten kann, ohne irgendeiner Gefährdung durch den Therapiestrahl 13 ausgesetzt zu sein. Man kann also von einem in Figur 1 erwähnten Raum 15, der links vom Zugang 17 angeordnet ist, ohne Weiteres bis zur Abschirmung 33 gelangen.

Aus einem hier ersichtlichen Strahlrohr 37 mit den üblichen Ablenk- und Scan-Komponenten, die hier im Therapieraum 7 angeordnet sind, tritt der Therapiestrahl 13 aus. Er ist auf den Behandlungsort 9 gerichtet, an dem hier eine Liege für einen Patienten vorgesehen ist. Durch einen Doppelpfeil 39 ist angedeutet, dass die Liege schwenkbar sein kann.

Aus dem Strahlrohr 37 und aus der in Richtung zum Behandlungsort 9 offenen Abschirmung 33 tritt der Therapiestrahl 13 aus und trifft auf den Behandlungsort 9. Jenseits des Behandlungsorts 9 gegebene Neutronenstrahlung 19 ist vom Zugang 17 weggerichtet und tritt durch die Wand 35 in das Erdreich 21 ein.

Bei dem in Figur 3 dargestellten Ausführungsbeispiel ist links und rechts der Abschirmung 33 - in Richtung des Therapiestrahls 13 gesehen - jeweils ein Labyrinth L vorgesehen, das dazu dient, vom Behandlungsort 9 reflektierte Strahlung abzufangen. Dadurch ist es möglich, den Zugang 17 ohne eine schwere Abschirmtür zu realisieren, was die Zugänglichkeit zum Behandlungsort 9 erleichtert und beschleunigt.

Bei dem hier dargestellten Ausführungsbeispiel ist noch vorgesehen, dass die Abschirmung 33 in ihrem dem Behandlungsort 9 gegenüberliegenden Bereich, der auch zum Zugang 17 weist, eine Abschirmverstärkung 41 vorzugsweise aus Eisen umfasst.

Die links- und rechts des Eintrittsbereichs E und der Abschirmung 33 liegenden Labyrinthe L innerhalb des Therapieraums 7 werden hier durch an der Innenseite der Wand 35 und an der Außenseite der Abschirmung 33 vorgesehene Stufen 43 und 45 realisiert. Denkbar ist es aber auch, hier in den Gang der Labyrinthe L vorspringende Wandabschnitte vorzusehen.

Aus der Draufsicht gemäß Figur 3 ist ersichtlich, dass der Therapieraum 7 symmetrisch ausgebildet ist, wobei auch die Abschirmung 33 den Eintrittsbereich E symmetrisch umgibt.

Die Abschirmung 33 weist zwei einander gegenüberliegende Wandbereiche 33a und 33b auf, zwischen denen der Eintrittsbereich E angeordnet ist. An dem dem Zugang 17 zugewandten Ende der Abschirmung 33 werden die Wandbereiche 33a, 33b durch einen Basisabschnitt 33c miteinander verbunden, sodass die im Wesentlichen U-förmige Ausgestaltung der Abschirmung 33 realisiert wird. Auf der dem Basisabschnitt 33c gegenüber liegenden Seite laufen sich die Wandbereiche 33a und 33b in Richtung auf den Behandlungsort 9 im Wesentlichen trichterförmig auseinander. Mit anderen Worten: Der zwischen den Wandbereichen liegende Bereich der Abschirmung 33 erweitert sich strahlabwärts von dem Eintrittsbereich E gesehen in Richtung auf den Behandlungsort 9, sodass sich ein trichterförmiger Auffangbereich für vom Behandlungsort 9 reflektierte Neutronenstrahlung ergibt. Diese Strahlung wird also optimal von der Abschirmung 33 abgefangen, sodass die Strahlenbelastung auf der dem Behandlungsort 9 gegenüber liegenden Seite der Abschirmung 33, also im Bereich des Zugangs 17 und sich daran anschließender Räume 15, auf ein Minimum reduziert wird.

Bei dem in Figur 3 dargestellten Ausführungsbeispiel steht die Abschirmung 33 frei im Therapieraum 7, sodass sich auf beiden Seiten ein Gang zwischen der Wand 35 des Therapieraums 7 und der Außenseite der Abschirmung 33 ergibt, nämlich die oben erwähnten Labyrinthe L. Bei diesem Ausführungsbeispiel des Behandlungsraums 7 ist vorgesehen, dass die dem Zugang 17 abgewandte und rechts vom Behandlungsort 9 angeordnete Begrenzungswand des Therapieraums 7 einen in das Erdreich 21 vorspringenden Bereich aufweist, sodass Bedienungspersonal leicht um den Behandlungsort 9 bzw. eine dort vorgesehene Liege für einen Patienten herumgehen kann. Dieser Vorsprung ist in seinen Abmessungen an den Raumbedarf anpassbar.

Figur 4 zeigt ein Ausführungsbeispiel eines Therapieraums 7, der gegenüber dem in Figur 3 dargestellten abgewandelt ist. Gleiche Teile sind mit gleichen Bezugsziffern versehen, sodass in sofern auf die Beschreibung zu Figur 3 verwiesen wird.

Der Therapieraum 7 gemäß Figur 4 ist etwas einfacher aufgebaut als der in Figur 3 dargestellte: Die dem Zugang 17 gegenüber liegende Begrenzungswand des Therapieraums 7 kann in einem etwas größeren Abstand zur Abschirmung 33 angeordnet sein, als in Figur 3 vorgesehen, sodass ausreichend Platz bleibt, um eine Patientenliege auf der dem Eintrittsort E gegenüber liegenden Seite herum zu gehen. Dafür ist die Begrenzungswand des Therapieraums hier gerade ausgebildet; es wird also auf den in Figur 3 dargestellten Vorsprung verzichtet, was die Herstellungskosten des Therapieraums 7 reduziert.

Auch hier ist aber das gleiche Grundprinzip realisiert: Der Hauptneutronenstrahlkegel, der vom Behandlungsort 9 nach rechts gerichtet ist, wird durch die Wand 35 des Therapieraums 7 in das Erdreich 21 geführt. Vom Behandlungsort 9 reflektierte Neutronenstrahlung wird von der Abschirmung 33 aufgefangen und gelangt damit allenfalls in äußerst abgeschwächter Form zum Zugang 17.

Im Übrigen ist der Innenaufbau des Therapieraums, insbesondere der der Abschirmung 33, identisch wie bei dem in Figur 3 dargestellten Ausführungsbeispiel, sodass auf die vorangegangen Erläuterungen verwiesen wird, um Wiederholungen zu vermeiden.

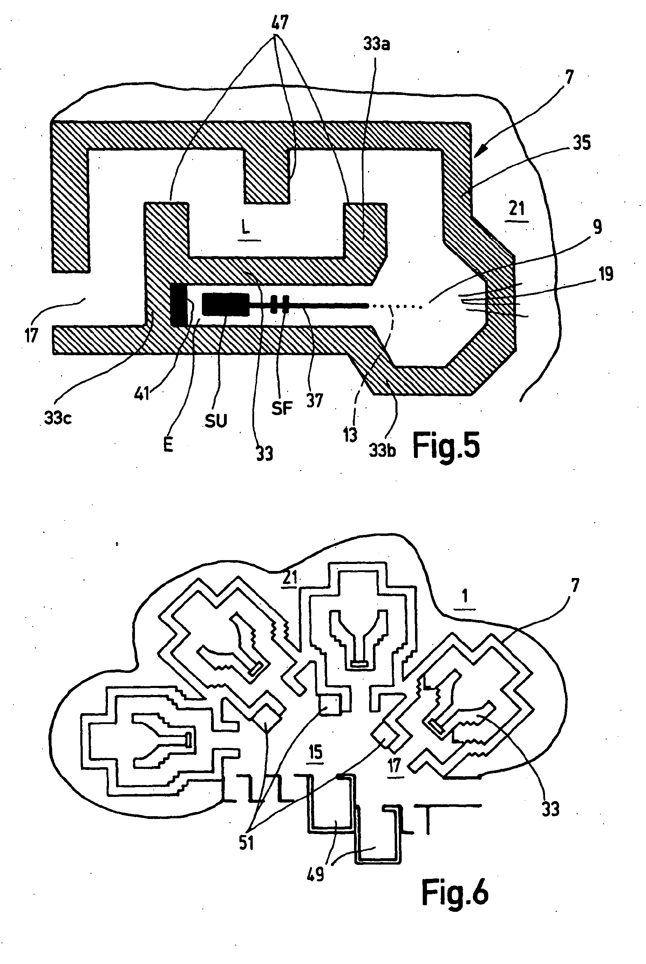

Figur 5 zeigt ein abgewandeltes Ausführungsbeispiel eines Therapieraums 7. Gleiche Teile sind mit gleichen Bezugsziffern versehen. Insofern wird zur Vermeidung von Wiederholungen auf die vorangegangene Beschreibung verwiesen.

Es wird deutlich, dass das zweite Ausführungsbeispiel des Therapieraums 7 asymmetrisch ausgebildet ist. Der Grundaufbau ist aber identisch:
Der Therapieraum 7 ist von einer Wand 35 umgeben, die aus üblichem Abschirmungsbeton oder dergleichen bestehen kann. Außerhalb des Therapieraums 7 befindet sich Erdreich 21. Links in Figur 5 ist der Zugang 17 zum Therapieraum 7 zu sehen. In einem Abstand zu diesem befindet sich der Eintrittsbereich E, in dem ein Teilstrahl T, wie er in den Figuren 1 bis 3 erläutert wurde, durch den Boden des Therapieraumes 7 in dessen Inneres eintritt und dort umgelenkt sowie geformt wird. Da der Teilstrahl T im Bereich des Zugangs 17 unterhalb des Bodens des Therapieraums 7 oder oberhalb der Decke verläuft, ist der Bereich zwischen Zugang 17 und Abschirmung 33 ungestört und ohne Gefährdung begehbar.

Der Therapiestrahl 13 wird durch ein übliches Strahlrohr 37 in Richtung auf den Behandlungsort 9 geleitet, wobei der Therapiestrahl 13 von dem Strahlrohr 37 aus gesehen nach rechts austritt, so dass der Zugang 17 in keiner Weise belastet wird. Im Bereich des Behandlungsorts 9 entstehende Neutronenstrahlung 19 ist nach rechts gerichtet und tritt durch die Wand 35 ins Erdreich 21, so dass hier auf eine besonders starke Abschirmung durch Beton oder dergleichen verzichtet werden kann.

Der Eintrittsbereich E ist durch eine zum Behandlungsort 9 offene Abschirmung 33 umgeben, wobei in Figur 4 oberhalb der Abschirmung 33 ein Labyrinth L geschaffen wird, das hier in den Gang des Labyrinths hineinragende Wandabschnitte 47 aufweist. Denkbar wäre es aber auch hier, Stufen vorzusehen, wie dies anhand von Figur 3 erläutert wurde.

Der Therapieraum 7 ist sehr kompakt ausgebildet, weil auf ein unterhalb der Abschirmung 33 gelegenes Labyrinth verzichtet und ein Teil der Abschirmung durch einen Bereich der Wand 35 gebildet wird.

Die Abschirmung 33 ist von ihrer Grundkonzeption also identisch wie die anhand von Figur 3 erläuterte: Sie weist einen oberen Wandbereich 33a und einen gegenüber liegenden unteren Wandbereich 33b auf. Zwischen diesen Wandabschnitten befindet sich der Eintrittsbereich E. Hier sind auch die Strahlumlenkungseinrichtung SU und die Strahlformungseinrichtung SF angeordnet. Die beiden Wandbereiche 33a und 33b sind durch einen Basisabschnitt 33c miteinander verbunden, sodass wiederum die U-förmige Abschirmung 33, wie oben beschrieben, gebildet wird. Auch hier ist im Bereich des Basisabschnitts 33c eine Abschirmungsverstärkung 41 vorgesehen, wie sie oben beschrieben wurde.

Die Abschirmung 33 ist also in Richtung auf den Zugang 17 geschlossen und in Richtung zum Behandlungsort 9 offen. Auch hier ist vorgesehen, dass die Wandbereiche 33a und 33b sich auf der dem Basisabschnitt 33c abgewandten Seite in Richtung auf den Behandlungsort 9 erweitern, sodass ein reflektierte Neutronenstrahlung auffangender Bereich geschaffen wird, wie er auch bei den Ausführungsbeispielen gemäß den Figuren 3 und 4 gegeben ist.

Ein Unterschied gegenüber den Ausführungsbeispielen nach den Figuren 3 und 4 ist darin zu sehen, dass ein Wandabschnitt der Abschirmung 33, hier der untere Wandbereich 33b, Teil der den Therapieraum 7 umgebenden Wand 35 ist, während der gegenüber liegende Wandbereich 33a frei im Therapieraum 7 liegt.

Auch bei dem in Figur 5 dargestellten Ausführungsbeispiel ist, wie bei den in den Figuren 1 bis 4 dargestellten Ausführungsformen ersichtlich, dass der Therapieraum 7 über den Zugang 17 frei zugänglich ist. Es bedarf hier keiner besonderen Abschirmungstür. Der Therapiestrahl 13 gelangt über den Eintrittsbereich E in den Therapieraum 7, wird dort umgelenkt und geformt und tritt über das Strahlrohr 37 aus und gelangt damit zum Behandlungsort 9. Dabei ist die Strahlrichtung vom Zugang 17 weggerichtet. Im Behandlungsort 9 entstehende Neutronenstrahlung 19 ist ebenfalls vom Zugang 17, damit auch von links vom Zugang 17 liegenden Bereichen, weggerichtet. Vom Behandlungsort 9 reflektierte Strahlung wird optimal von der Abschirmung 33, insbesondere auch wogen deren trichterförmigen Aufweitung in Richtung zum Behandlungsort 9, gegenüber dem Zugang 17 abgeschirmt.

Allen Ausführungsbeispielen ist gemeinsam, dass ein räumlich begrenzter Teil des Therapieraums 7 vom Eintrittsbereich E bis zum Behandlungsort 9 und ein strahlabwärts vom Behandlungsort 9 liegender Bereich, der von Neutronenstrahlung 19 belastet ist, sehr gut gegen Strahlung abgeschirmt ist. Die dazu eingesetzte Abschirmung 33 umgibt den Eintrittsbereich E beidseitig, außerdem die Strahlumlenkungseinrichtung SU und die Strahlformungseinrichtung SF. Darüber hinaus schützt die Abschirmeinrichtung 33 vor reflektierter Neutronenstrahlung, sodass die übrigen Bereiche des Therapieraums einer sehr stark reduzierten Strahlenbelastung ausgesetzt sind. Dadurch, dass der Therapiestrahl 13 von oben oder unten im Eintrittsbereich E in den Therapieraum gelangt, sind auf der dem Behandlungsorten 9 gegenüberliegenden Seite der Abschirmung 33 freie Bereiche vorhanden, nämlich der Zugang 17, über den der Therapieraum ungestört betreten werden kann.

Der Therapieraum 7 ist also optimal nutzbar, weil vom Zugang 17 bis zur Abschirmung 33 keine störenden Bauteile vorhanden sind. Im Übrigen ist sichergestellt, dass der Therapiestrahl 13 vom Zugang 17 aus gesehen weggerichtet ist in Richtung zum Behandlungsort 9. Auf einer gedachten Verbindungslinie zwischen dem Zugang 17 und dem Behandlungsort 9 liegt die Abschirmung 33, wobei eben der Therapiestrahl 13 und damit vom Behandlungsort 9 ausgehende Neutronenstrahlung 19 vom Zugang weggerichtet sind. Es bedarf also im Bereich des Zugangs 17 und sich dort nach links anschließender Räumlichkeiten keiner weiteren besonderen Abschirmungsmaßnahmen mehr. Im Eintrittsbereich E entstehende Strahlung und vom Behandlungsort 9 reflektierte Neutronenstrahlung werden von der Abschirmung 33 optimal abgefangen.

Auch bei dem in Figur 5 dargestellten Ausführungsbeispiel tritt also der Therapiestrahl 13 nicht durch eine Seitenwand sondern durch den Boden in den Therapieraum 7 ein. Grundsätzlich wäre auch eine Einleitung durch die Decke möglich. Strahlführungseinrichtungen, wie sie anhand der Figuren 1 und 2 erläutert wurden, befinden sich also unter- oder oberhalb des Therapieraums 7 und stören daher den Zugang 17 nicht, der weder vom Therapiestrahl 13 noch von der Neutronenstrahlung 19 betroffen ist. Im Bereich des Behandlungsorts 9 entstehende Streustrahlung wird von der Abschirmung 33 und von dem Labyrinth L so effektiv abgefangen, dass auf schwere Abschirmtüren im Bereich des Zugangs 17 verzichtet werden kann. Anhand der Figuren 6 bis 9 sollen verschiedene Anordnungsmöglichkeiten von Therapieräumen erläutert werden:

Figur 6 zeigt eine Anzahl von symmetrisch aufgebauten Therapieräumen 7, wie sie in den Figuren 2, 3 und 4 bereits erläutert wurden. Sie sind vorzugsweise alle identisch ausgebildet und weisen symmetrisch zur Mittelachse des Therapieraums 7 angeordnete Labyrinthe L auf, die eine Verbindung zwischen dem Zugang 17 und dem Behandlungsort 9 schaffen und rechts und links an der Abschirmung 33 vorbeiführen.

In allen Fällen wurde auf die Darstellung des Strahlrohrs 37 verzichtet, weil es hier lediglich auf die Anordnung der Therapieräume 7 ankommt. Diese sind möglichst dicht aneinander gelegt, so dass sich quasi Berührungspunkte und gemeinsame Wandbereiche ergeben. Die Zugänge 17 aller Therapieräume 7 führen zu einem Raum 15, der zur Vorbereitung und Versorgung von Patienten dient und auch Röntgenbereiche 49 und Lagerungsräume für Patienten umfassen kann sowie Schalträume 51 für medizinisch technische Assistenten, die die Bestrahlungseinrichtung 1, insbesondere die den einzelnen Therapieräumen 7 zugeordneten Geräte, bedienen und überwachen. Die Röntgenbereiche 49 dienen insbesondere der Lageverifizierung von Patienten. Dabei wird hier ausdrücklich darauf hingewiesen, dass Röntgendiagnostik zur Lageverifizierung auch in den Therapieräumen 7 durchführbar ist.

Die Therapieräume 7 sind gemäß Figur 6 bogenförmig angeordnet, um den gemeinsamen Raum 15 optimal nutzen zu können, und um den Raumbedarf insgesamt für die Therapieräume 7 auf ein Minimum zu beschränken. Diese sind, wie durch eine Linie angedeutet, auch hier von Erdreich 21 umgeben, so dass strahlabwärts von dem Behandlungsort 9 gegebene Neutronenstrahlung 19 von Erdreich 21 abgefangen werden und auf teure Abschirmungen hierfür verzichtet werden kann.

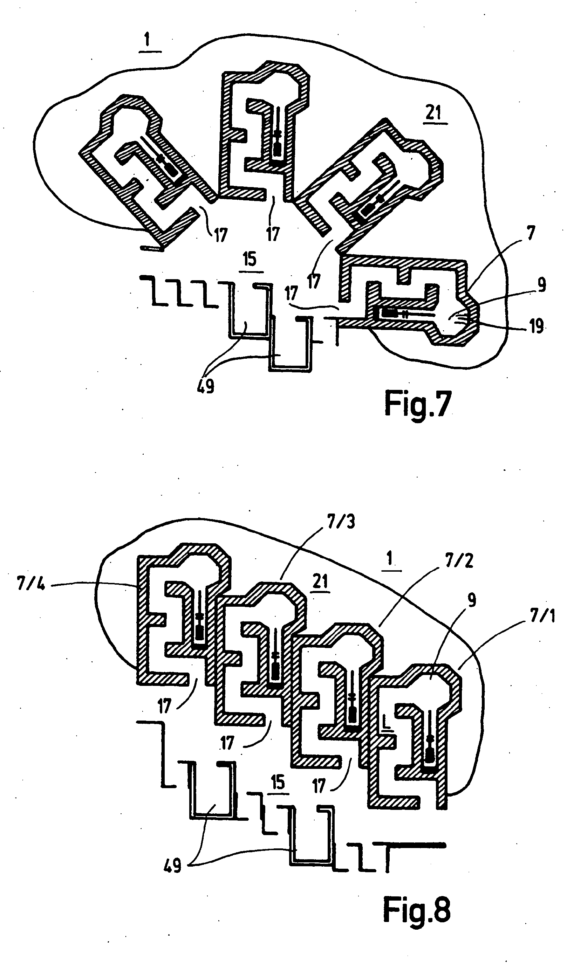

Figur 7 zeigt ein anderes Ausführungsbeispiel einer Anordnung von Therapieräumen 7. Es sind hier Therapieräume bogenförmig angeordnet, wie sie anhand von Figur 5 erläutert wurden. Es sind hier also, anders als in Figur 6, asymmetrische Behandlungsräume bogenförmig angeordnet, wobei ihre Zugänge zu einem gemeinsamen Raum 15 führen, in dem Patienten der Vor- und Nachsorge unterworfen werden und liegen können. Außerdem können auch hier Röntgenbereiche 49 vorgesehen werden sowie hier nicht im Einzelnen wiedergegebene Schalträume zur Bedienung der Bestrahlungseinrichtung 1 und zur Durchführung der Behandlung von Patienten in den Therapieräumen 7.

Auch bei dem hier dargestellten Ausführungsbeispiel sind, wie in Figur 2 und 6, die Therapieräume 7 so angeordnet, dass die Therapiestrahlen 13 und Neutronenstrahlung 19 nach außen weggerichtet sind und auf das die Therapieräume 7 umgebende Erdreich 21 treffen. Der Raum 15 wird weder durch den Therapiestrahl 13 der einzelnen Therapieräume noch durch Neutronenstrahlung, die im Bereich der Behandlungsorte 9 entstehen kann, belastet.

Figur 8 zeigt schließlich eine Bestrahlungseinrichtung 1 mit einer Anzahl von Therapieräumen 7, wie sie anhand von Figur 5 erläutert wurden. Es handelt sich also hier um asymmetrische Therapieräume 7 mit nur einem Labyrinth L.

Aus der Darstellung gemäß Figur 8 ist, wie aus Figur 5 ersichtlich, dass im Bereich des Behandlungsorts 9 quasi eine Auswölbung gegeben ist, dass aber im Übrigen die Therapieräume 7 parallel zueinander verlaufende Seitenwände aufweisen. Bei der Darstellung gemäß Figur 8 sind die Therapieräume 7 parallel zueinander angeordnet und in Längsrichtung zueinander versetzt, so dass die parallelen Wandbereiche zweier benachbarter Therapieräume jeweils aneinander liegen und somit ein minimaler Raumbedarf gegeben ist.

Die Zugänge 17 der Therapieräume 7 führen auch hier zu einem Raum 15, der Röntgenbereiche 49 oder hier nicht im Einzelnen dargestellte Schalträume umfassen kann.

Auch hier ist die Anordnung und Ausrichtung der Therapieräume 7 so gewählt, dass die Therapiestrahlen 13 und Neutronenstrahlung 19 von dem Zugang 17 und dem Raum 15 weggerichtet ist und von dem die Therapieräume umgebenden Erdreich 21 abgeschirmt wird.

Da auch hier die Therapiestrahlen 13 und die Neutronenstrahlung 19 den Zugang 17 nicht belasten, kann auf Abschirmtüren verzichtet werden, so dass ein leichter und rascher Zugang zu den Behandlungsorten 9 möglich ist. Allerdings ist bei den asymmetrischen Therapieräumen 7 davon auszugehen, dass das Labyrinth L nur wechselweise als Zu- beziehungsweise Ausgang verwendet werden kann.

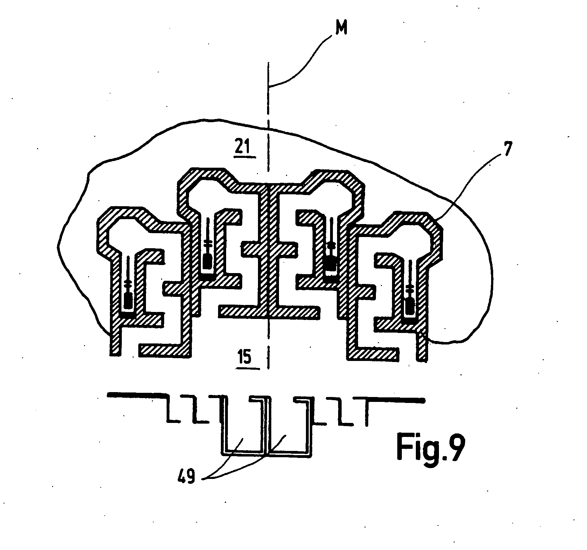

In Figur 9 wird schließlich noch ein Ausführungsbeispiel dargestellt, bei dem vier Therapieräume 7 einer Bestrahlungseinrichtung 1 dargestellt sind. Diese sind spiegelbildlich zu einer gedachten Mittelebene M angeordnet, wobei auch die Therapieräume rechts und links der Mittelebene M spiegelbildlich ausgebildet sind: Bei den rechts der Mittelebene M liegenden Therapieräumen befindet sich das Labyrinth L links von der Abschirmung 33 des Eintrittsbereichs E, bei den links der Mittelebene M liegenden Therapieräumen rechts davon. Die Zugänge 17 der Therapieräume führen wiederum zu einem gemeinsamen Raum 15, in dem Patienten vor- und nachbehandelt werden können. Auch hier sind Röntgenbereiche 49 zu erkennen. Es können auch hier Schalträume 51 vorgesehen werden, die hier allerdings nicht dargestellt sind.

Auch hier sind die Therapieräume 7 raumsparend dicht an dicht angeordnet, wobei jeweils sichergestellt ist, dass die Therapiestrahlen 13 und die Neutronenstrahlung 19 die Zugänge 17 und den gemeinsamen Raum 15 nicht treffen. Vielmehr ist die Neutronenstrahlung 19 jeweils nach außen gerichtet und wird von dem die Therapieräume 7 umgebenden Erdreich 21 abgefangen.

Insgesamt zeigt sich, dass die Bestrahlungseinrichtung 1 unterschiedliche Therapieräume 7 aufweisen kann, die auf verschiedene Art und Weise zueinander angeordnet werden können. Dabei können symmetrisch aufgebaute Therapieräume mit zwei Labyrinthen und asymmetrisch aufgebaute Therapieräume mit jeweils einem Labyrinth eingesetzt werden. Auch ist es - nach Bedarf - sehr wohl möglich, symmetrische und asymmetrische Therapieräume zu kombinieren, um eine optimale Raumausnutzung zu gewährleisten. Auf jeden Fall ist gewährleistet, dass zumindest die Therapieräume 7 und die Strahlführungseinrichtungen 5 auf unterschiedlichen Ebenen angeordnet sind, so dass die Zugänge 17 zu den Therapieräumen 7 optimal angeordnet werden können. Die Strahlen werden jeweils in einen Abstand zu den Begrenzungswänden eines Therapieraums 7 in dessen Inneres eingeleitet, so dass jeweils innerhalb der Therapieräume 7 ein Eintrittsbereich E durch eine eigene Abschirmung 33 umgeben werden kann und der Zugang 17 vor dem Therapiestrahl 13 und der Neutronenstrahlung 19 geschützt ist.

Insgesamt zeigt sich, dass die Bestrahlungseinrichtung 1 sehr kompakt aufgebaut ist und dass die Verteilung von Therapieraum 7, Strahlungsquelle 3 und/oder Strahlführungseinrichtung 5 auf unterschiedliche Ebenen quasi eine Verschachtelung der Elemente der Bestrahlungseinrichtung 1 erlaubt, was zu einer sehr kompakten Bauform führt. Darüber hinaus sind die Therapieräume 7 der Bestrahlungseinrichtung 1 so ausgebildet, dass der Strahl nicht durch eine Seitenwand des Therapieraums 7 sondern durch den Boden B oder die Decke desselben in den Innenraum eingeleitet und auf den Behandlungsort 9 gerichtet werden kann. Dadurch ist es möglich, den Zugang 17 so zu legen, dass dieser weder durch den Therapiestrahl 13 noch durch Neutronenstrahlung 19 belastet ist und ein Labyrinth L zwischen dem Zugang 17 und dem Behandlungsort 9 geschaffen werden kann. Dieses kann symmetrisch zum Eintrittsbereich E des Strahls in den Therapieraum 7 ausgebildet sein und einen Zu- und einen Ausgang aufweisen. Noch kompakter werden die Therapieräume, wenn nur ein einziges Labyrinth L vorgesehen wird, das sowohl als Zu- als auch als Ausgang dient.

Dadurch, dass die Therapiestrahlen 13 und insbesondere die Neutronenstrahlung 19 vom Zugang 17 und von dem Zugang vorgeordneten Räumen 15 weggerichtet ist, kann die Bestrahlungseinrichtung 1 dicht an ein Behandlungs- oder Forschungszentrum sowie an eine Klinik 23 herangelegt werden. Dabei entfallen teure Abschirmungsmaßnahmen, weil der Therapiestrahl 13 und die Neutronenstrahlung 19 in das den Therapieraum 7 umgebende Erdreich 21 dringen und von diesem abgefangen werden.

Derartig ausgebildet Therapieräume 7 können dicht aneinander bogenförmig oder parallel zueinander angeordnet werden, wobei auch eine versetzte Aneinanderreihung von Therapieräumen 7 möglich ist. Damit kann auch mehreren Therapieräumen 7 ein gemeinsamer Raum 15 zugeordnet werden, was die Raumnutzung weiter optimiert.

Besonders aus den Figuren 8 und 9 wird deutlich, dass bei der hier gezeigten Anordnung von Therapieräumen Wandabschnitte dünner ausgebildet werden oder ganz entfallen können, um Abschirmmaterial und Raum zu sparen. Dies gilt insbesondere in den Bereichen, in denen Wandabschnitte benachbarter Therapieräume aneinander liegen. Hier ergibt sich bei herkömmlichen Einrichtungen oft eine Wandstärke, die die zur gewünschten Abschirmung erforderliche Dicke überschreitet.

## Patentansprüche

1. Bestrahlungseinrichtung (1) für die Protonen- und/oder Ionenstrahltherapie mit einer Strahlungsquelle (3), einer Strahlführungseinrichtung (5) und mit mindestens einem, wenigstens einen Behandlungsort (9) und einen Zugang (17) umfassenden Therapieraum (7), in den ein Therapiestrahl (13) eingeleitet wird, wobei der Therapieraum (7) in einer ersten Ebene (E1) angeordnet ist, und der Therapiestrahl (13) aus einer ober- oder unterhalb der ersten Ebene liegenden zweiten Ebene (E2) in den Therapieraum (7) eingeleitet, dort umgelenkt und auf den Behandlungsort (9) so gerichtet wird, dass der auf den Behandlungsort (9) gerichtete Therapiestrahl (13) vom Zugang (17) weggerichtet ist, wobei im Therapieraum eine dem Eintrittsbereich (E) des Therapiestrahls (13) in den Therapieraum (7) zugeordnete, in Richtung auf den Behandlungsort (9) offene Abschirmung (33) vorgesehen ist, wobei der Zugang (17) auf der dem Behandlungsort (9) abgewandten Seite der Abschirmung (33) angeordnet ist, und seitlich versetzt zu dem im Therapieraum (7) verlaufenden Therapiestrahl (13) und zur Abschirmung (33) mindestens ein vom Zugang (17) zum Behandlungsort (9) führendes Labyrinth (L) vorgesehen ist.

2. Bestrahlungseinrichtung nach Anspruch 1, wobei der im Eintrittsbereich (E) in den Therapieraum (7) eintretende Therapiestrahl (13) einer Strahlumlenkungseinrichtung (SU) und/oder einer Strahlformungseinrichtung (SF) zugeführt wird, die im Therapieraum (7) angeordnet sind/ist.

3. Bestrahlungseinrichtung nach Anspruch 1 oder 2, wobei der Therapiestrahl (13) bis zum Eintrittsbereich (E) unter- oder oberhalb des Zugangs (17) verläuft.

4. Bestrahlungseinrichtung nach einem der vorhergehenden Ansprüche, wobei die Abschirmung (33) zwei Wandbereiche (33a, 33b) umfasst, zwischen denen der Eintrittsbereich (E) liegt, von denen einer Teil einer Abschirmwand (35) des Therapieraums (7) sein kann.

5. Bestrahlungseinrichtung nach einem der vorhergehenden Ansprüche, wobei die Strahlumlenkungseinrichtung (SU) und/oder die Strahlformungseinrichtung (SF) zwischen den Wandbereichen (33a, 33b) der Abschirmung (33) angeordnet sind/ist.

6. Bestrahlungseinrichtung nach Anspruch 3, wobei die Abschirmung (33) einen die Wandbereiche (33a, 33b) verbindenden Basisabschnitt (33c) aufweist.

7. Bestrahlungseinrichtung nach Anspruch 6, wobei der Basisabschnitt (33c) eine Abschirmverstärkung (41) aufweist.

8. Bestrahlungseinrichtung nach Anspruch 7, wobei die Wandbereiche (33a, 33b) der Abschirmung (33) sich strahlabwärts von dem Eintrittsbereich (E) gesehen in Richtung auf den Behandlungsort (9) erweitern.

9. Bestrahlungseinrichtung nach einem der vorhergehenden Ansprüche, wobei die Abschirmung (33) symmetrisch aufgebaut ist.

10. Bestrahlungseinrichtung nach einem der vorhergehenden Ansprüche, wobei zwischen Zugang (17) und Behandlungsort (9) auf beiden Seiten der Abschirmung (33) ein Labyrinth (L) vorgesehen ist.

11. Bestrahlungseinrichtung, nach einem der vorhergehenden, Ansprüche, wobei mindestens ein Raum (15) zur Vor- und Nachbehandlung von am Behandlungsort (9) behandelten Patienten und für den Aufenthalt von sonstigen Personen auf der ersten Ebene (E1) angeordnet ist.

12. Bestrahlungseinrichtung nach Anspruch 11, wobei der aus dem Eintrittsbereich (E) auf den Behandlungsort (9) gerichtete Therapiestrahl (13) von dem mindestens einen Raum (15) weggerichtet ist.

13. Bestrahlungseinrichtung nach einem der vorhergehenden Ansprüche, wobei der Therapiestrahl (13) unter einem Winkel auf der Behandlungsort (9) gerichtet ist.

14. Bestrahlungseinrichtung nach einem der Ansprüche 1 bis 12, wobei der Therapiestrahl (13) parallel zum Boden (B) des Therapieraums (7) verläuft.

15. Bestrahlungseinrichtung nach einem der vorhergehenden Ansprüche, wobei die Strahlungsquelle (3) in einer dritten Ebene (E3) angeordnet ist.

16. Bestrahlungseinrichtung nach einem der vorhergehenden Ansprüche, wobei mehrere Therapieräume (7/1,7/2,7/3,7/4) bogenförmig zueinander angeordnet sind.

17. Bestrahlungseinrichtung nach einem der vorhergehenden Ansprüche, wobei mehrere Therapieräume (7) parallel zueinander angeordnet sind.

18. Bestrahlungseinrichtung nach Anspruch 17, wobei die Therapieräume zueinander versetzt angeordnet sind.

19. Bestrahlungseinrichtung nach einem der vorhergehenden Ansprüche, wobei die Therapieräume zu einer gedachten Mittelebene (M) gespiegelt angeordnet und/oder ausgebildet sind.

## Claims

1. Irradiation device (1) for proton and/or ion radiotherapy, with a radiation source (3), a beam-guiding device (5) and with at least one therapy room (7), which comprises at least one treatment site (9) and an access (17), and into which a therapy beam (13) is introduced, wherein the therapy room (7) is arranged on a first level (E1) and the therapy beam (13) is introduced into the therapy room (7) from a second level (E2), lying above or below the first level, deflected there and directed at the treatment site (9) such that the therapy beam (13) directed at the treatment site (9) is directed away from the access (17), wherein provision is made for a shielding (33) in the therapy room, which shielding is associated with the entry region (E) of the therapy beam (13) into the therapy room (7) and is open in the direction of the treatment site (9), wherein the access (17) is arranged on that side of the shielding (33) facing away from the treatment site (9), and provision is made for at least one labyrinth (L), which leads from the access (17) to the treatment site (9) and is laterally offset with respect to the therapy beam (13) running in the therapy room (7) and with respect to the shielding (33).

2. Irradiation device according to Claim 1, wherein the therapy beam (13) entering the therapy room (7) in the entry region (E) is fed to a beam deflection device (SU) and/or a beam shaping device (SF), which are/is arranged in the therapy room (7).

3. Irradiation device according to Claim 1 or 2, wherein the therapy beam (13) runs below or above the access (17) up until the entry region (E).

4. Irradiation device according to one of the preceding claims, wherein the shielding (33) comprises two wall regions (33a, 33b), between which the entry region (E) is located and one of which can be part of a shielding wall (35) of the therapy room (7).

5. Irradiation device according to one of the preceding claims, wherein the beam deflection device (SU) and/or the beam shaping device (SF) are/is arranged between the wall regions (33a, 33b) of the shielding (33).

6. Irradiation device according to Claim 3, wherein the shielding (33) has a base section (33c) that connects the wall regions (33a, 33b).

7. Irradiation device according to Claim 6, wherein the base section (33c) has a strengthening (41) of the shielding.

8. Irradiation device according to Claim 7, wherein the wall regions (33a, 33b) of the shielding (33) widen in the direction of the treatment site (9) when viewed downstream of the entry region (E).

9. Irradiation device according to one of the preceding claims, wherein the shielding (33) has a symmetric design.

10. Irradiation device according to one of the preceding claims, wherein provision is made for a labyrinth (L) on both sides of the shielding (33) between access (17) and treatment site (9).

11. Irradiation device according to one of the preceding claims, wherein at least one room (15) for pre- and post-treatment of patients treated at the treatment site (9) and for other persons to stay is arranged on the first level (E1).

12. Irradiation device according to Claim 11, wherein the therapy beam (13) directed at the treatment site (9) from the entry region (E) is directed away from the at least one room (15).

13. Irradiation device according to one of the preceding claims, wherein the therapy beam (13) is directed at the treatment site (9) at an angle.

14. Irradiation device according to one of Claims 1 to 12, wherein the therapy beam (13) runs parallel to the floor (B) of the therapy room (7).

15. Irradiation device according to one of the preceding claims, wherein the radiation source (3) is arranged on a third level (E3).

16. Irradiation device according to one of the preceding claims, wherein a plurality of therapy rooms (7/1, 7/2, 7/3, 7/4) are arranged in an arc shape with one another.

17. Irradiation device according to one of the preceding claims, wherein a plurality of therapy rooms (7) are arranged in parallel to one another.

18. Irradiation device according to Claim 17, wherein the therapy rooms are arranged offset to one another.

19. Irradiation device according to one of the preceding claims, wherein the therapy rooms are arranged and/or designed such that they are mirrored in an imagined central plane (M).

## Revendications

1. Dispositif d'irradiation (1) pour la thérapie par rayonnement protonique et/ou ionique, qui comporte une source de rayonnement (3), un dispositif de guidage des rayons (5) et au moins une chambre de thérapie (7) comprenant au moins un lieu de traitement (9) et un accès (17) et dans laquelle est introduit un rayon de thérapie (13), cette chambre (7) étant disposée dans un premier plan (E1) et le rayon de thérapie (13) étant introduit, à partir d'un second plan (E2) situé au-dessus ou au-dessous du premier plan, dans la chambre où il est alors dévié et dirigé vers le lieu de traitement (9) dans le sens opposé à l'accès (17), et dans la chambre de thérapie est prévu, associé à la zone d'entrée (E) du rayon de thérapie (13) dans la chambre (7), un écran (33) ouvert en direction du lieu de traitement (9), l'accès (17) se trouvant du côté de l'écran (33) opposé à la chambre de traitement (9) et il est prévu, décalé par rapport au rayon de thérapie (13) passant dans la chambre de thérapie (7) et par rapport à l'écran (33), au moins un labyrinthe (L) conduisant de l'accès (17) au lieu de traitement (9).

2. Dispositif d'irradiation selon la revendication 1, dans lequel le rayon de thérapie (13) qui entre dans la chambre de thérapie est amené, dans la zone d'entrée (E) à un dispositif de déviation de rayon (SU) et/ou à un dispositif de formage de rayon (SF), disposé(s) dans la chambre de thérapie (7).

3. Dispositif d'irradiation selon la revendication 1 ou 2, dans lequel le rayon de thérapie (13), jusqu'à la zone d'entrée (E), circule au-dessus et au-dessous de l'accès (17).

4. Dispositif d'irradiation selon une des revendications précédentes, dans lequel l'écran (33) comprend deux zones de paroi (33a, 33b) entre lesquelles se trouve la zone d'entrée (E) et qui peuvent être une partie d'une paroi d'écran (35) de la chambre de thérapie (7).

5. Dispositif d'irradiation selon une des revendications précédentes, dans lequel le dispositif de déviation de rayon (SU) et/ou le dispositif de formage de rayon (SF) est (sont) disposé(s) entre les zones de paroi (33a, 33b) de l'écran (33).

6. Dispositif d'irradiation selon la revendication 3, dans lequel l'écran (33) présente une partie de base (33c) qui relie les zones de paroi (33a, 33b).

7. Dispositif d'irradiation selon la revendication 6, dans lequel la partie de base (33c) présente un renforcement d'écran (41).

8. Dispositif d'irradiation selon la revendication 7, dans lequel les zones de paroi (33a, 33b) de l'écran (33) observées depuis la zone d'entrée (E) dans le sens aval du rayonnement vont en s'élargissant en direction du lieu de traitement (9).

9. Dispositif d'irradiation selon une des revendications précédentes, dans lequel l'écran (33) est symétrique.

10. Dispositif d'irradiation selon une des revendications précédentes, dans lequel, entre l'accès (17) et le lieu de traitement (9), il est prévu un labyrinthe (L) des deux côtés de l'écran (33).

11. Dispositif d'irradiation selon une des revendications précédentes, dans lequel sur le premier plan (E1) se trouve au moins un espace (15) pour le pré ou post traitement de patients affectés au lieu de traitement (9) et pour accueillir d'autres personnes.

12. Dispositif d'irradiation selon la revendication 11, dans lequel le rayon de thérapie (13), dirigé de la zone d'entrée (E) vers le lieu de traitement (9) est écarté d'au moins un espace (15).

13. Dispositif d'irradiation selon une des revendications précédentes, dans lequel le rayon de thérapie (13) est dirigé selon un angle sur le lieu de traitement (9).

14. Dispositif d'irradiation selon une des revendications 1 à 12, dans lequel le rayon de thérapie (13) est parallèle au sol (B) de la chambre de thérapie (7).

15. Dispositif d'irradiation selon une des revendications précédentes, dans lequel la source de rayonnement (3) se trouve dans un troisième plan (E3).

16. Dispositif d'irradiation selon une des revendications précédentes, dans lequel plusieurs chambres de thérapie (7/1, 7/2, 7/3) sont disposées en formant un arc.

17. Dispositif d'irradiation selon une des revendications précédentes, dans lequel plusieurs chambres de thérapie (7/1, 7/2, 7/3) sont disposées parallèlement.

18. Dispositif d'irradiation selon la revendication 17, dans lequel les chambres de thérapie sont décalées entre elles.

19. Dispositif d'irradiation selon une des revendications précédentes, dans lequel les chambres de thérapie sont disposées et/ou réalisées symétriquement par rapport à un plan médian (M) virtuel.
